# EUROPEAN PATENT APPLICATION

(11) **EP 2 404 610 A1**
(43) Date of publication of application: **11.01.2012**
(21) Application number: 10748786.0
(22) Date of filing: 03.03.2010
(51) Int. Cl.: A61K 38/00, A61P 19/00, A61P 19/02, A61P 43/00, C07K 14/74, C12N 15/09

(54) **Prophylactic or therapeutic agent for rheumatoid arthritis or rheumatoid arthritis-related diseases**

(30) Priority: 03.03.2009 JP 2009049792
(71) Applicant: Kyushu University, National University Corporation, Fukuoka-shi Fukuoka 812-8581 (JP)
(72) Inventor: MAENAKA, Katsumi, Fukuoka-shi Fukuoka 812-8581 (JP); KUROKI, Kimiko, Fukuoka-shi Fukuoka 812-8581 (JP); OHDO, Shigehiro, Fukuoka-shi Fukuoka 812-8581 (JP); KOYANAGI, Satoru, Fukuoka-shi Fukuoka 812-8581 (JP)
(74) Representative: Vossius & Partner
(86) International application number: PCT/JP2010/053459
(87) International publication number: WO 2010/101192

(57) **Abstract**

Disclosed is a prophylactic or therapeutic agent for rheumatoid arthritis or diseases caused by rheumatoid arthritis (rheumatoid arthritis-related diseases), which comprises HLA-G or a dimer thereof as an active ingredient. As the HLA-G, the following protein (a) or (b) are suitably used.
(a) A protein consisting of the amino acid sequence of SEQ ID NO: 1
(b) A protein consisting of the amino acid sequence of SEQ ID NO: 1 with the deletion, substitution, or addition of one or several amino acids excluding the amino acid at position 42, and having a binding activity for leukocyte Ig-like receptors and/or CD8

## Description

### Technical Field

The present invention relates to prophylactic or therapeutic agents for rheumatoid arthritis or for diseases caused by rheumatoid arthritis (rheumatoid arthritis-related diseases).

### Background Art

HLA-G, a human nonclassical major histocompatibility antigen (Major Histocompatibility Complex, MHC), is expressed tissue specifically unlike the classical MHC class I molecules. Specifically, HLA-G is known to have an important role during pregnancy, namely, expression in the fetus trophoblast on the placenta, and involvement in immunosuppression preventing the fetus from being recognized as a foreign matter and attacked by the maternal immune system. Based on these findings, HLA-G has been used in many different applications. For example, there have been attempts to use HLA-G or genetically modified HLA-G molecules in fertility treatment, suppression of rejections in organ transplantation, and autoimmune disease therapy.

The HLA-G receptors reported so far include CD8 molecules expressed mainly on T cells, leukocyte Ig-like receptors B1/B2 (LILRB1/LILRB2; also known as Ig-like transcripts (ILT2/ILT4), LIR1/LIR2, and CD85j/CD85d) expressed in a wide range of immune system cells, and KIR2DL4. The immunosuppressive effect of HLA-G is believed to be mediated by LILRB1 and LILRB2. The HLA-G and receptor binding has been analyzed in detail, for example, through the studies of interactions between the LILR family (LILRB1 and LILRB2) and HLA-G, as previously reported by the present inventors (Non-Patent Literature 1).

It is known that HLA-G molecules exist as dimers through natural oxidation. The dimers are formed by formation of the intermolecular disulfide bond between the free cysteine residues of HLA-G molecules. Studies by the present inventors have revealed that the dimer more strongly binds to the immunosuppressive receptor groups and thus has much higher signaling capability than the HLA-G monomer (hereinafter, simply "HLA-G" or "monomer"). Specifically, the signaling capability of the dimer has been found to be about 100-fold higher than the signaling capability of the monomer (Non-Patent Literature 2, Patent Literature 1).

The HLA-G dimer molecules are present in the body as are HLA-G molecules. The HLA-G dimer is thus expected for providing safe anti-inflammatory agents with few side effects (Patent Literature 1). It is, however, generally agreed among skilled artisans that, because of the wide range of diseases covered by such anti-inflammatory agents, the actual efficacy cannot be determined unless in vivo experiments are conducted.

### Citation List

### Patent Literature

PTL 1: WO 2007/011044

### Non-Patent Literature

NPL 1: M. Shiroishi et al., Proc. Natl. Acad. Sci. USA., National Academy of Sciences, 2003, Vol.100, No.15, p8856-8861)
NPL 2: M. Shiroishi et al., Journal of Biological Chemistry, The American Society for Biochemistry and Molecular Biology, Inc., 2006, Vol.281, No.15, p.10439-10447

### Summary of Invention

### Technical Problem

It is an object of the present invention to provide novel medicinal uses of the HLA-G or HLA-G dimer. More specifically, an object of the present invention is to provide novel medicinal uses of the HLA-G or HLA-G dimer as a prophylactic or therapeutic agent for rheumatoid arthritis and for diseases caused by rheumatoid arthritis (rheumatoid arthritis-related diseases).

### Solution to Problem

The present inventors conducted intensive studies to solve the foregoing problems, and produced new findings.

Specifically, the present inventors administered a HLA-G (monomer) and a dimer thereof to type-II collagen-induced rheumatoid arthritis model mice, and found that both the monomer and the dimer produce excellent anti-rheumatoid arthritis activity, and that the dimer exhibits the anti-rheumatoid arthritis activity at lower concentrations compared to the monomer. Further, by comparing the effect of the monomer administered for five successive days and of the monomer administered once on the first day (single administration), it was found that a single administration is sufficient to obtain the anti-rheumatoid arthritis effect. A similar investigation for the dimer revealed that its anti-rheumatoid arthritis effect can last at least about two months from the single administration date. The present inventors also found that the anti-rheumatoid arthritis effect can be obtained throughout the four limbs even with a local administration (local intracutaneous administration to the left foot). From these findings, the present inventors confirmed that the HLA-G or HLA-G dimer (preferably, the dimer) is useful as an active ingredient of an anti-rheumatoid arthritis agent that can sustainably exhibit excellent efficacy in small amounts.

The present invention was completed based on these findings, and includes the following aspects.

### (I) Prophylactic or Therapeutic Agent for Rheumatoid Arthritis or

### Rheumatoid Arthritis-Related Disease

(I-1) A prophylactic or therapeutic agent for rheumatoid arthritis or for a disease caused by rheumatoid arthritis (hereinafter, also referred to as "rheumatoid arthritis-related disease"), the prophylactic or therapeutic agent including a HLA-G or a dimer thereof as an active ingredient. The prophylactic or therapeutic agent contains a HLA-G or a dimer thereof in an effective amount with which the prophylactic or therapeutic agent can exhibit anti-rheumatoid arthritis activity, specifically, in an effective proportion for the prevention or treatment of rheumatoid arthritis or rheumatoid arthritis-related diseases.

### (1-2) A prophylactic or therapeutic agent according to (I-1), wherein the HLA-G is a protein of (a) or (b) below:

(a) a protein consisting of the amino acid sequence of SEQ ID NO: 1,
(b) a protein consisting of the amino acid sequence of SEQ ID NO: 1 with the deletion, substitution, or addition of one or several amino acids excluding the amino acid at position 42, and having a binding activity for leukocyte Ig-like receptors and/or CD8.

(1-3) A prophylactic or therapeutic agent of (I-1) or (I-2), wherein the HLA-G dimer is a dimer with an intermolecular disulfide bond that links the HLA-G between cysteine residues at position 42 of a HLA-G amino acid sequence.

(I-4) A prophylactic or therapeutic agent according to any one of (I-1) to (I-3), wherein the prophylactic or therapeutic agent is a preparation that includes the HLA-G (monomer) as an active ingredient, and that is of a form locally administered to a patient with rheumatoid arthritis or with a rheumatoid arthritis-related disease, or to a patient having the possibility of developing the disease, once in at least two months, preferably once in half a year.

(I-5) A prophylactic or therapeutic agent according to any one of (I-1) to (I-3), wherein the prophylactic or therapeutic agent is a preparation that includes the HLA-G dimer as an active ingredient, and that is of a form locally administered to a patient with rheumatoid arthritis or with a rheumatoid arthritis-related disease, or to a patient having the possibility of developing the disease, once in at least three months, preferably once in two months.

### (II) Prophylactic or Therapeutic Method for Rheumatoid Arthritis or

### Rheumatoid Arthritis-Related Disease

(II-1) A method for preventing or treating rheumatoid arthritis or a rheumatoid arthritis-related disease, the method including the step of administering an effective amount of a composition that includes a HLA-G or a dimer thereof as an active ingredient to a patient with rheumatoid arthritis or with a rheumatoid arthritis-related disease, or to a patient having the possibility of developing the disease. As used herein, "composition" has a form of preferably a pharmaceutical preparation. Further, as used herein, "effective amount" means an effective amount for the treatment of rheumatoid arthritis or a rheumatoid arthritis-related disease, or an effective amount for the prevention of the onset of the disease.

### (II-2) A method according to (II-1), wherein the HLA-G is a protein of (a) or (b) below:

(a) a protein consisting of the amino acid sequence of SEQ ID NO: 1,
(b) a protein consisting of the amino acid sequence of SEQ ID NO: 1 with the deletion, substitution, or addition of one or several amino acids excluding the amino acid at position 42, and having a binding activity for leukocyte Ig-like receptors and/or CD8.

(II-3) A method according to (I-1) or (1-2), wherein the HLA-G dimer is a dimer with an intermolecular disulfide bond that links the HLA-G between cysteine residues at position 42 of a HLA-G amino acid sequence.

(II-4) A method according to any one of (II-1) to (II-3), wherein the composition is a pharmaceutical preparation that includes the HLA-G (monomer) as an active ingredient, and is locally administered to a patient with rheumatoid arthritis or with a rheumatoid arthritis-related disease, or to a patient having the possibility of developing the disease, once in at least two months, preferably once in half a year.

(II-5) A method according to any one of (II-1) to (II-3), wherein the composition is a pharmaceutical preparation that includes the HLA-G dimer as an active ingredient, and is locally administered to a patient with rheumatoid arthritis or with a rheumatoid arthritis-related disease, or to a patient having the possibility of developing the disease, once in at least three months, preferably once in two months.

### (III) Use for the Prevention or Treatment of Rheumatoid Arthritis or Rheumatoid Arthritis-Related Disease

(III-1) A HLA-G, a dimer thereof, or a pharmaceutical composition for use in the prevention or treatment of rheumatoid arthritis or a disease caused by rheumatoid arthritis;
the pharmaceutical composition comprising the HLA-G or the HLA-G dimer in an effective proportion for the prevention or treatment of rheumatoid arthritis or a rheumatoid arthritis-related disease.

(III-2) A HLA-G or a dimer thereof, or a pharmaceutical composition including the HLA-G or the HLA-G dimer according to (III-1), wherein the HLA-G is a protein of (a) or (b) below.

(III-3) A HLA-G or a dimer thereof, or a pharmaceutical composition including the HLA-G or the HLA-G dimer according to (III-1) or (III-2), wherein the HLA-G dimer is a dimer with an intermolecular disulfide bond that links the HLA-G between cysteine residue at position 42 of a HLA-G amino acid sequence.

(III-4) A HLA-G or a dimer thereof, or a pharmaceutical composition including the HLA-G or the HLA-G dimer according to any one of (III-1) to (III-3), wherein the pharmaceutical composition is a preparation that includes the HLA-G (monomer) as an active ingredient, and that is of a form locally administered to a patient with rheumatoid arthritis or with a rheumatoid arthritis-related disease, or to a patient having the possibility of developing the disease, once in at least three months, preferably once in half a year.

(III-5) A HLA-G or a dimer thereof, or a pharmaceutical composition including the HLA-G or the HLA-G dimer according to any one of (III-1) to (III-3), wherein the pharmaceutical composition is a preparation that includes the HLA-G dimer as an active ingredient, and that is of a form locally administered to a patient with rheumatoid arthritis or with a rheumatoid arthritis-related disease, or to a patient having the possibility of developing the disease, once in at least two months, preferably once in three months.

### (IV) Use for Production of Prophylactic or Therapeutic Agent for Rheumatoid Arthritis or Rheumatoid Arthritis-Related Disease

(IV-1) A use of a HLA-G or a dimer thereof for the production of a prophylactic or therapeutic agent for rheumatoid arthritis or a rheumatoid arthritis-related disease. The prophylactic or therapeutic agent contains a HLA-G or a dimer thereof in an effective amount with which the prophylactic or therapeutic agent can exhibit anti-rheumatoid arthritis activity, specifically, in an effective proportion for the prevention or treatment of rheumatoid arthritis or rheumatoid arthritis-related diseases.

(IV-2) A use according to (IV-1), wherein the HLA-G is a protein of (a) or (b) below.

(IV-3) A use according to (IV-1) or (IV-2), wherein the HLA-G dimer is a dimer with an intermolecular disulfide bond that links the HLA-G between cysteine residues at position 42 of a HLA-G amino acid sequence.

(IV-4) A use according to any one of (IV-1) to (IV-3), wherein the prophylactic or therapeutic agent is a preparation that includes the HLA-G (monomer) as an active ingredient, and that is of a form locally administered to a patient with rheumatoid arthritis or with a rheumatoid arthritis-related disease, or to a patient having the possibility of developing the disease, once in at least three months, preferably once in half a year.

(IV-5) A use according to any one of (IV-1) to (IV-3), wherein the prophylactic or therapeutic agent is a preparation that includes the HLA-G dimer as an active ingredient, and that is of a form locally administered to a patient with rheumatoid arthritis or with a rheumatoid arthritis-related disease, or to a patient having the possibility of developing the disease, once in at least two months, preferably once in three months.

### Advantageous Effects of Invention

The present invention can provide a novel medicinal use of a HLA-G or a dimer thereof, specifically, a novel medicinal use as a prophylactic or therapeutic agent for rheumatoid arthritis or diseases caused by rheumatoid arthritis (rheumatoid arthritis-related diseases). The active ingredient of the prophylactic or therapeutic agent for rheumatoid arthritis or rheumatoid arthritis-related diseases of the present invention is a HLA-G or a dimer thereof, a component naturally present in the body of humans. The prophylactic or therapeutic agent of the present invention thus has few side effects and high safety. Further, the prophylactic or therapeutic agent of the present invention can exhibit a sustained anti-rheumatoid arthritis effect for at least 20 days with the active ingredient HLA-G, and for at least two months with the active ingredient HLA-G dimer, in a small, single local administration to a patient with rheumatoid arthritis or with a rheumatoid arthritis-related disease, or to a patient having the possibility of developing the disease. The prophylactic or therapeutic agent of the present invention is also safe in this respect, and represents a pharmaceutical preparation that is less demanding to a patient.

### Brief Description of Drawings

FIG. 1 is a schematic diagram representing a procedure for producing HLA-G-coding modified genes ((b) HLA-GEC gene, (c) HLA-GQCa gene, (d) HLA-GQCb gene, (e) HLA-GQCab gene); the procedure includes the steps of producing HLA-GEC gene (b) from the wild-type HLA-G gene (a) by total synthesis, and producing HLA-GQCa gene (c), HLA-GQCb gene (d), and HLA-GQCab gene (e) from HLA-GEC gene (b); the solid region (dark region) in HLA-GQCa gene (c), HLA-GQCb gene (d), and HLA-GQCab gene (e) means a modified region.

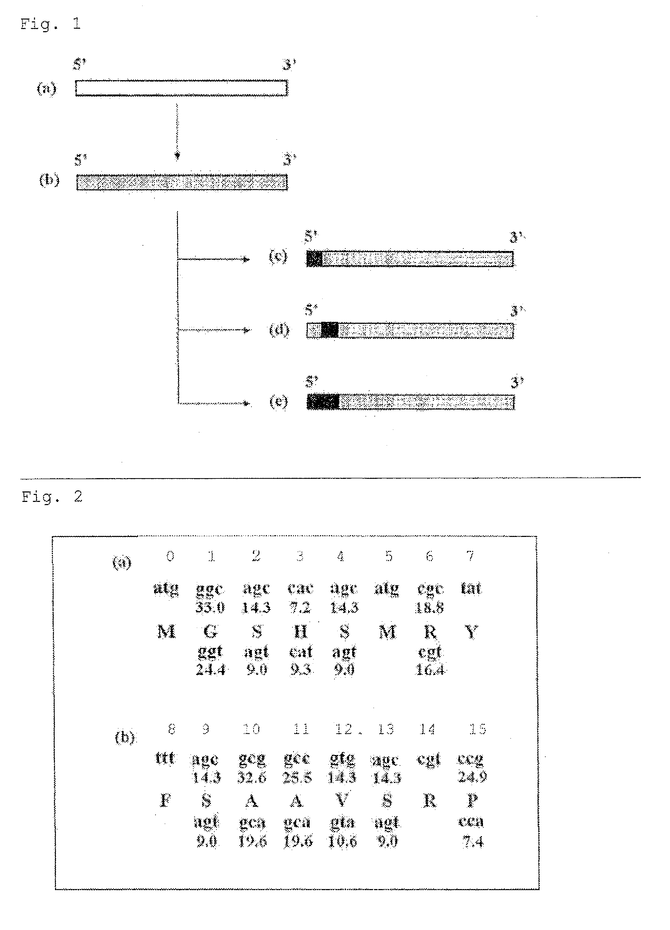

FIG. 2 in (a) represents the amino acid sequence from residue 0 (M: Met) to residue 7 (Y: Tyr) of a construct that includes Met added to the N-terminal of the wild-type HLA-G (residue 0 being the methionine (Met) at the N-terminal), and the corresponding codons (the upper codons are the codons of the wild-type HLA-G gene, the lower codons are the codons of modified HLA-G gene), the sequence corresponding to the solid region in FIG. 1, (c); FIG. 2 in (b) represents the amino acid sequence from residue 8 (F: Phe) to residue 15 (P: Pro) of the wild-type HLA-G, and the corresponding codons (the upper codons are the codons of the wild-type HLA-G gene, the lower codons are the codons of modified HLA-G gene), the sequence corresponding to the solid region in FIG. 1, (d).
FIG. 3 is a diagram representing the electrophoretic patterns of bacteria for the HLA-G genes of FIG. 1 ((a) HLA-G gene, (b) HLA-GEC gene, (c) HLA-GQCa gene, (d) HLA-GQCb gene, (e) HLA-GQCab gene).
FIG. 4 is a diagram representing the response of LILRB2 (LIR2; dotted line) to HLA-G (solid line) or BSA (negative control).
FIG. 5 is a diagram representing the Kd value (4.1 µM) of HLA-G and LILRB2 (LIR2).
FIG. 6 represents the results of the observation for the presence or absence of HLA-G dimer formation by gel filtration chromatography using Superdex 200 10/300; D, a dimer peak; M, a monomer peak; the vertical axis, absorbance at wavelength 280 nm (Absorbance at 280 nm [mAD]); the horizontal axis, elution volume (ml); A, incubation for 10 days without DTT (4°C) ; B, incubation for 3 days with 2 mM DTT (4°C).
FIG. 7 represents the criteria of RA score: (a) the evidence of arthritis at the large joint of limbs, and (b) the evidence of arthritis at the small joint of fingers.
FIG. 8 represents the experiment results for a rheumatoid arthritis early onset group (150 µg, 15 µg, 1.5 µg administration), and the experiment results for a rheumatoid arthritis late onset group (150 µg, 15 µg, 1.5 µg administration).
FIG. 9 represents changes in RA score with time after the continuous administration of a HLA-G monomer (monomer; solid circle) and PBS (control; solid triangle) to the rheumatoid arthritis onset group for 5 days (Experiment Example 2).

### Description of Embodiments

The present invention relates to a prophylactic or therapeutic agent for rheumatoid arthritis or rheumatoid arthritis-related diseases, which includes HLA-G or a dimer thereof as an active ingredient, and to use of such prophylactic or therapeutic agents.

### (1) Active Ingredient

### (1-1) HLA-G (monomer)

HLA-G is a nonclassical MHCI molecule with some unique properties not found in classical MHCIs:
(i) Very limited expression only in a few tissues, such as extravillous trophoblast, thymic epithelial cells, and some tumors (J. LeMaoult, et al., (2003) Tissue Antigens, 62, 273-84.)
(ii) Limited polymorphism
(iii) Slow transport
(iv) Relatively limited peptide presentation

HLA-G molecules bind to inhibitory receptors such as leukocyte Ig-like receptors (LILR) to inhibit the immune response of a wide range of immune cells, including myeloid monocytes, T cells, and NK cells, and induce immune tolerance.

The HLA-G concerned with the present invention is preferably a human-derived HLA-G. Information, including the amino acid sequence (SEQ ID NO: 1) and the coding base sequence (SEQ ID NO: 2) of HLA-G is known, and is available from, for example, NCBI with Accession Number: AF226990.

The HLA-G concerned with the present invention is not limited to the HLA-G (wild-type HLA-G), and may be a protein (mutated) of the amino acid sequence (SEQ ID NO: 1) of the wild-type HLA-G with the deletion, substitution, or addition of one or several amino acids, provided that it has the function of the wild-type HLA-G, specifically the binding activity for leukocyte Ig-like receptors (LILRB1 and LILRB2) and/or CD8.

As used herein, "protein (mutated) of the amino acid sequence with the deletion, substitution, or addition of one or several amino acids" means a protein (mutated) that has the function of the wild-type HLA-G (binding activity for leukocyte Ig-like receptors and/or CD8), and that includes a deletion, substitution, or addition of amino acids with preferably 95% or more identity with the amino acid sequence (SEQ ID NO: 1) of the wild-type HLA-G. More preferably, the amino acid sequence of the protein (mutated; hereinafter, "mutated HLA-G") has 96% or more identity, further preferably 98% or more identity with the amino acid sequence (SEQ ID NO: 1) of the wild-type HLA-G.

The mutation sites in the amino acid sequence (SEQ ID NO: 1) of the wild-type HLA-G for the mutated HLA-G must exclude the amino acid residues (Vall94, Phe195, Asp196, Tyr197, Glu198, Thor200, Gln226, Asp227, Glu229, Va1248) considered important for the binding reaction with the leukocyte Ig-like receptors and/or CD8.

Further, because the presence of the cysteine residue (Cys42) that contributes to the intermolecular disulfide bonds of HLA-G is important for the formation of a HLA-G dimer, the mutated HLA-G preferably has a mutation (deletion, substitution, or addition) at sites other than the amino acid residue 42 in the amino acid sequence (SEQ ID NO: 1) of the wild-type HLA-G.

As used herein, the "binding activity for leukocyte Ig-like receptors and/or CD8" means the direct binding activity of the HLA-G for leukocyte Ig-like receptors and/or CD8, capable of exhibiting immuno-control effects through leukocyte Ig-like receptor- or CD8-mediated signaling.

The binding activity of the mutated HLA-G for leukocyte Ig-like receptors and/or CD8 can be confirmed by, for example, a reporter assay using T cell hybridoma. An example of the T cell hybridoma is an NFAT-GFP introduced reporter cell (mouse T cell hybridoma) expressing a chimeric molecule as a fused molecule of the LILRB1 or LILRB2 extracellular domain and the transmembrane/intracellular domain of activation receptor PILRb. In this case, the HLA-G upon binding to the LILR receptor induces signaling through the PILRb intracellular domain, and activates the transcription factor NFAT. The reporter assay is an assay system that takes advantage of the induction of GFP expression through the NFAT activation. GFP expression indicates the binding of LILRBs and HLA-G, and can be confirmed by checking the GFP fluorescence using flow cytometry.

The HLA-G can be prepared as follows. First, a recombinant vector is constructed by incorporating a HLA-G amino acid sequence-coding gene into an expression vector or the like using a known gene recombinant technique. The recombinant vector is then introduced into a host to obtain a transformant using known transformation procedures. The transformant is cultured, and the expressed recombinant HLA-G is collected.

The gene that encodes the HLA-G amino acid sequence may be a gene (SEQ ID NO: 2) that encodes the amino acid sequence (SEQ ID NO: 1) of the wild-type HLA-G, or a gene that encodes the amino acid sequence of the mutated HLA-G.

The gene that encodes the mutated HLA-G may be prepared by introducing mutations to the DNA sequence of the wild-type HLA-G gene. For example, the gene may be prepared according to the site-directed mutation introducing method described in Molecular Cloning, A Laboratory Manual 2nd ed., Cold Spring Harbor Laboratory Press (1989), Current Protocols in Molecular Biology, John Wiley & Sons (1987-1997). Specifically, the gene may be prepared with a site-directed mutation introducing kit, using known techniques such as the Kunkel or Gapped duplex technique. Preferred examples of such kits include QuickChange^{™} Site-Directed Mutagenesis Kit (Stratagene), GeneTailarl^{™} Site-Directed Mutagenesis System (Invitrogen), and TaKaRa Site-Directed Mutagenesis System (Mutan-K, Mutan-Super Express Km, etc.; Takara Bio).

The host used to prepare the transformant is not particularly limited, as long as it can express HLA-G (wild-type HLA-G, mutated HLA-G) from the introduced recombinant vector or the like. For example, the host may be known host cells, including cells derived from animals such as humans and mice, cells derived from various insects, prokaryotic cells such as *Escherichia coli*, eukaryotic cells such as yeasts, and plant cells.

A problem of using *Escherichia coli* as the host is the extremely low HLA-G (wild-type HLA-G, mutated HLA-G) expression efficiency (an expression product yield of 2 to 3 mg per 1-L liquid culture). This problem can be solved with the use of a HLA-G amino acid sequence-coding gene (hereinafter, "modified gene") modified to make all the codons encoding the total amino acids of the HLA-G (wild-type HLA-G, mutated HLA-G) suitable for expression in *Escherichia coli,* and to include a specific base sequence in a region downstream of the start codon (a region from residue 0 to 15 of a construct that has Met added to the N terminal of the wild-type HLA-G (residue 0 being the methionine (Met) at the N terminal)). Specifically, the HLA-G gene modified to make all the codons encoding the total amino acids of the wild-type HLA-G suitable for expression in *Escherichia coli* may have the base sequence of SEQ ID NO: 3. The modified gene may be further modified by (a) substituting the positions 1 to 18 (corresponding to the base sequence that encodes the amino acids 1 to 6 in the amino acid sequence of HLA-G) of the base sequence (SEQ ID NO: 3) with the base sequence of SEQ ID NO: 4, and/or (b) substituting the positions 25 to 45 (corresponding to the base sequence that encodes the amino acids 9 to 15 in the amino acid sequence of HLA-G) of the base sequence (SEQ ID NO: 3) with the base sequence of SEQ ID NO: 5. The base sequences of the modified gene after the modification (a), (b), and (a) and (b) to the base sequence of SEQ ID NO: 3 are represented in SEQ ID NOS: 6, 7, and 8, respectively.

With the modified gene, as much as about 30 mg more HLA-G per 1-L liquid culture can be collected even when *Escherichia coli* is used as a host (see Preparation Example 1).

Specifically, the recombinant HLA-G can be produced by a method that includes the steps of culturing the transformant, and collecting the recombinant HLA-G from the resulting culture. As used herein, "culture" means any of a culture supernatant, cultured cells, cultured bacteria, and disrupted cells or bacteria. The transformant may be cultured using methods commonly used for culturing hosts. The target protein accumulates in the culture.

When the recombinant HLA-G is produced outside of the cells, the liquid culture is used either directly or by removing the cells by, for example, centrifugation or filtration. This is followed by, for example, extraction by ammonium sulfate precipitation, which is performed as required to collect the recombinant HLA-G from the culture. The recombinant HLA-G can then be isolated and purified by using dialysis or various chromatography techniques (such as gel filtration, ion exchange chromatography, and affinity chromatography), as required.

When the recombinant HLA-G is produced within the cells, the recombinant HLA-G may be collected by disrupting the cells. When the HLA-G is contained in the soluble fraction, the disrupted cell residues (including the cell extract insoluble fraction) are removed, if need be, by, for example, centrifugation or filtration after the disruption. The supernatant after the residue removal is the cell extract soluble fraction, and can be obtained as a coarsely purified protein solution. On the other hand, when the HLA-G is expressed as an inclusion body in the insoluble fraction, the insoluble fraction is isolated by centrifugation after the disruption, and the disrupted cell residues are removed by repeated washing and centrifugation with a buffer that contains a surfactant or the like. The resulting inclusion body is solubilized with a denaturant-containing buffer (such as a guanidine or urea buffer), and the protein is unwound by dilution or dialysis. The functionally unwound HLA-G can be isolated and purified using various chromatography techniques (such as gel filtration, ion exchange chromatography, and affinity chromatography).

In addition to the protein synthesis system using a transformant, an acellular protein synthesis system that does not make use of any viable cells also can be used to produce the recombinant HLA-G. The recombinant HLA-G so produced may be purified by a means appropriately selected from techniques such as chromatography.

### (1-2) HLA-G Dimer

The HLA-G dimer is a dimeric form of HLA-G forming a crosslinked structure between the cysteine residues at position 42 in the amino acid sequence of the HLA-G monomer, specifically a dimer with an intermolecular disulfide bond formed by the oxidation of the thiol group (SH group) in the cysteine residues (disulfide-linked dimer; see WO2007/011044).

The HLA-G dimer is not limited; it may be a homodimer of the wild-type HLA-G or of the mutated HLA-G, or a heterodimer of the wild-type HLA-G and the mutated HLA-G. Preferably, the HLA-G dimer is a homodimer of the wild-type HLA-G. It should be noted that, when the HLA-G monomer as the constituting unit of the HLA-G dimer is the mutated HLA-G, the cysteine residue that contributes to forming the intermolecular disulfide bond means a cysteine residue that corresponds to the residue 42 in the amino acid sequence of the wild-type HLA-G.

Further, the HLA-G dimer has a structure with the disulfide bond portion situated inside, and the binding site for leukocyte Ig-like receptors and/or CD8 exposed to outside. Owning to this structure, there is no steric hinderance for receptor binding, and the HLA-G dimer can maintain the functions of the HLA-G monomer.

Preferably, the HLA-G dimer has, for example, a two-fold or higher binding efficiency for leukocyte Ig-like receptors and/or CD8 than the HLA-G monomer. Binding efficiency can be measured by, for example, a reporter assay using T cell hybridoma, a surface plasmon resonance analysis, or a native gel shift assay. Because of the very high binding efficiency for leukocyte Ig-like receptors and/or CD8, the HLA-G dimer can be effectively used to suppress the immune system cell activities.

Any method can be used to obtain the disulfide-linked HLA-G dimer, and the method of obtaining the HLA-G dimer is not particularly limited. For example, the HLA-G dimer may be a dimer obtained by a known method of naturally forming a disulfide bond, or a dimer formed by a method that can promote formation of a disulfide bond, as will be described later.

An example of a method that promotes formation of a disulfide bond is a method that adds a reducing agent to the HLA-G monomer. The reducing agent may be, for example, dithiothreitol (DTT), β-mercaptoethanol, reduced glutathione, or cysteamine, of which DTT is particularly preferable. The reducing agent attacks the thiol group in the cysteine residue that contributes to the disulfide bond formation in the HLA-G dimerization reaction, and promotes formation of the intermolecular disulfide bond between the thiol groups through oxidation reaction.

The reaction solvent used for the HLA-G dimerization reaction is not limited, and, for example, an aqueous solvent that contains components such as Tris-HCl (pH 8.0) and NaCl at appropriate concentrations is preferably used. Further, in the HLA-G dimer production, it is preferable that the HLA-G monomer be present in high concentrations, for example, 0.1 to 10 mg/mL, preferably 5 to 10 mg/mL, particularly preferably about 10 mg/mL. For example, the HLA-G may be concentrated by concentrating a HLA-G lysate.

In the foregoing method, the reducing agent is added preferably in small amounts with respect to the HLA-G monomer, for example, at a final concentration of 0.1 to 10 mM, preferably 1 to 5 mM. When DTT is used as the reducing agent, the particularly preferred final concentration is about 2 mM. The small addition of the reducing agent is particularly preferred for the HLA-G monomer present in high concentrations, because it can maximize the production efficiency of the HLA-G dimer.

### (2) Preparation Form

The active ingredient HLA-G or HLA-G dimer in the prophylactic or therapeutic agent of the present invention (hereinafter, collectively referred to as "preparation of the present invention", the preparation being one form of a composition (pharmaceutical composition) concerned with the present invention) may be used in the form of, for example, various salts and hydrates, as required. Further, the HLA-G or a dimer thereof may be used in the state of being chemically modified in a suitable fashion, taking into consideration preservation stability (particularly for maintaining activity). Further, the HLA-G or a dimer thereof may be used in a crystalline state or a dissolved state.

The preparation of the present invention may contain other components, in addition to the active ingredient HLA-G or HLA-G dimer. Examples of other components include various pharmaceutical components (various pharmaceutically acceptable carriers, etc.) as may be required depending on the use (used form) of the preparation. These other components may be appropriately contained to such an extent that the addition of these components does not impair the effects exhibited by the active ingredient of the present invention (therapeutic effects for rheumatoid arthritis or rheumatoid arthritis-related diseases, and the effect of preventing the onset of these diseases).

In the preparation of the present invention, the mixed proportion of the active ingredient HLA-G or HLA-G dimer is not limited, and these active ingredients are preferably mixed in the effective amounts for the treatment of rheumatoid arthritis or rheumatoid arthritis-related diseases, or for preventing the onset of these diseases. For example, when the active ingredient is HLA-G, the content is preferably 0.01 weight% or more, more preferably 1 weight% or more, further preferably 10 weight% or more with respect to the total preparation. When the active ingredient is a HLA-G dimer, the content is preferably 0.01 weight% or more, more preferably 1 weight% or more, further preferably 10 weight% or more with respect to the total preparation. The anti-rheumatoid arthritis activity of the present invention can be exhibited in these proportions of the active ingredient.

Further, the active ingredient HLA-G or HLA-G dimer is preferably purified as much as possible. Specifically, for example, the purity is preferably 50% or more, more preferably 80% or more, further preferably 90% or more.

The preparation of the present invention may be administered to humans or non-human mammals in various administration routes, specifically, by oral or parenteral administration (for example, intravenous injection, intramuscular injection, subcutaneous administration, intracutaneous administration, rectal administration, and transdermal administration). The preferred administration route is parenteral local administration, such as subcutaneous administration and transdermal administration.

Accordingly, the active ingredient HLA-G or HLA-G dimer, in addition to be used alone, may be prepared into a suitable dosage form for different administration routes by using a pharmaceutically acceptable carrier according to a common method.

Examples of preferred oral dosage forms include tablets, powders, subtle granules, granules, coated tablets, capsule formulations, syrups, and troches. Examples of preferred parenteral form include inhalation, suppositories, injections (including drops), ointments, eye-drops, eye ointments, nasal preparations, eardrops, gel patches, lotions, and liposome agents.

Examples of carriers usable for the preparation of these preparations include common excipients, binders, disintegrants, lubricants, colorants, flavoring agents, and, as required, stabilizers, emulsifiers, absorption promoters, surfactants, pH adjusters, antiseptics, anti-oxidizing agents, bulking agents, wetting agents, surface activating agents, dispersants, buffers, preservatives, solubilizing agents, and soothing agents. These carriers may be mixed with known components that can be used as raw material of drug preparations, and formed into preparations using an ordinary method.

Examples of nontoxic components usable in the preparation of the present invention include animal and plant oils such as soybean oil, beef tallow, and synthetic glyceride; hydrocarbons such as liquid paraffin, squalane, and solid paraffin; ester oils such as octyldodecyl myristate, and isopropyl myristate; higher alcohols such as cetostearyl alcohol, and behenyl alcohol; silicon resin; silicon oil; surfactants such as polyoxyethylene fatty acid ester, sorbitan fatty acid ester, glycerine fatty acid ester, polyoxyethylene sorbitan fatty acid ester, polyoxyethylene hydrogenated castor oil, and polyoxyethylene-polyoxypropylene block copolymers; water-soluble polymers such as hydroxyethyl cellulose, polyacrylic acid, carboxyvinyl polymer, polyethyleneglycol, polyvinylpyrrolidone, and methylcellulose; lower alcohols such as ethanol and isopropanol; polyalcohols (polyols) such as glycerine, propylene glycol, dipropylene glycol, sorbitol, and polyethyleneglycol; sugars such as glucose and sucrose; inorganic powders such as silicic anhydride, magnesium aluminum silicate, and aluminum silicate; inorganic salts such as sodium chloride, and sodium phosphate; and purified water. These may be used as salts or hydrates.

Preferred examples of excipients include lactose, fructose, cornstarch, sucrose, glucose, mannitol, sorbit, crystalline cellulose, and silicon dioxide. Preferred examples of binders include polyvinyl alcohol, polyvinyl ether, methylcellulose, ethylcellulose, gum arabic, tragacanth, gelatin, shellac, hydroxypropyl methylcellulose, hydroxypropylcellulose, polyvinylpyrrolidone, block polymers of polypropyleneglycol and polyoxyethylene, and meglumine. Preferred examples of disintegrants include starch, agar, gelatin powder, crystalline cellulose, calcium carbonate, sodium bicarbonate, calcium citrate, dextrin, pectin, and carboxymethylcellulose calcium. Preferred examples of lubricants include magnesium stearate, talc, polyethylene glycol, silica, and hydrogenated vegetable oil. The colorant may be those cleared for use in drugs. Preferred examples of flavoring agents include a cocoa powder, menthol, an aromatic powder, mint oil, borneol, and a cinnamon powder. These may be used as salts or hydrates.

When the preparation of the present invention is an oral formulation, the active ingredient HLA-G or HLA-G dimer may be formed into, for example, a powder, a subtle granule, a granule, a tablet, a coated tablet, or a capsule formulation by using an ordinary method, after adding an excipient and, as required, components, for example, such as a binder, a disintegrant, a lubricant, a colorant, and a flavoring agent. In the case of tablets and granules, the preparation may be, for example, sugar-coated, or may be appropriately coated using other known techniques, as required. In the case of syrup and injection preparations, the preparation may be formed using an ordinary method, after adding, for example, a pH adjuster, a solubilizer, a tonicity agent, and, as required, components such as a solubilizing agent and a stabilizer. In the case of topical agents, the method used to form the preparation is not limited, and an ordinary method can be used. A variety of raw materials commonly used in applications such as in drugs, quasi drugs, and cosmetics can be used as the base raw material. Examples of such materials include animal and plant oil, mineral oil, ester oil, waxes, higher alcohols, fatty acids, silicon oil, surfactants, phospholipids, alcohols, polyalcohols, water-soluble polymers, clay minerals, and purified water. Components such as pH adjusters, anti-oxidizing agents, chelating agents, preservatives and fungicides, colorants, and fragrances may be added, as required. Further, components such as blood flow increasing agents, disinfectants, antiphlogistics, cell stimulants, vitamins, amino acids, moisturizers, and keratolytic agents may be mixed, as required. In this case, the proportion of the HLA-G or a dimer thereof with respect to the carrier is not limited, and may be appropriately set within the range of from 1 to 90 weight%.

### (3) Target Disease: Rheumatoid Arthritis or Rheumatoid Arthritis-Related Disease

The preparation of the present invention is used for alleviating or relieving symptoms of rheumatoid arthritis, or for the prevention of diseases caused by rheumatoid arthritis (rheumatoid arthritis-related diseases).

As used herein, "rheumatoid arthritis" refers to a progressive autoimmune disease characterized by synovial joint inflammations throughout the body. An early symptom of the disease is joint pain, which progresses into joint deformation, or damages in body organs such as in blood vessels, heart, lungs, skin, and muscles.

The American College of Rheumatology (ACR) classification criteria below are generally used for the diagnosis of rheumatoid arthritis:
1. Morning stiffness (lasting at least 1 hour)
2. Multiple arthritis (at least three joint areas have swelling)
3. Swelling of hand joints
4. Symmetric joint swelling
5. Rheumatoid nodule
6. Positive for rheumatoid factor (RF)
7. Typical evidence on joint radiographs

A diagnosis for rheumatoid arthritis can be made when at least four out of these seven criteria are met.

As used herein, "anti-rheumatoid arthritis activity" or "anti-rheumatoid arthritis effect" means the activity or effect for reducing at least Item 3 (hand joint swelling) in the foregoing seven criteria, preferably swelling at the large joints of the hands. Further, the "anti-rheumatoid arthritis activity" or "anti-rheumatoid arthritis effect" as used herein includes the activity or effect for alleviating the progression of rheumatoid arthritis symptoms by suppressing symptoms of arthritis.

Further, as used herein, "rheumatoid arthritis-related disease" means a disease caused by rheumatoid arthritis, in other words, a disease that occurs with the progression of rheumatoid arthritis symptoms. The onset of rheumatoid arthritis-related disease can be prevented ("prevent" as used herein includes delaying the onset of disease), or the extent of the disease onset can be relieved by alleviating or relieving the progression of rheumatoid arthritis symptoms.

Specific examples of rheumatoid arthritis-related disease include ophthalmologic diseases such as Sjogren's syndrome, keratoconjunctivitis sicca (including dry eye), rheumatoid nodule, scleromalacia perforans, episcleritis, and scleritis; respiratory diseases such as interstitial pneumonia, bronchiolitis obliterans, pleurisy, pneumothorax, pyothorax, airway lesion, pleural lesion, rheumatic nodule, vascular lesion, and sleep apnea (temporomandibular joint lesion, cricoarytenoid joint lesion); heart diseases such as epicarditis, symptomatic epicarditis, chronic constrictive pericarditis, valve dysfunction, embolism, conductive disturbance, myocardial damage, aortitis, aortic regurgitation, and aneurysm rupture; digestive tract diseases such as AA amyloidosis caused by chronic inflammation, and ischemic enteritis caused by rheumatoid vasculitis; kidney diseases such as interstitial nephritis caused by Sjogren's syndrome in association with rheumatoid arthritis, interstitial renal lesion, proteinurine, secondary amyloidosis, and glomerular lesion (membranous nephropathy) caused by AA amyloidosis in association with rheumatoid arthritis; neurological diseases such as spinal cord damage caused by cervical deformation, compression neuropathy caused by tenosynovitis, and mononeuropathy multiplex caused by vasculitis in association with rheumatoid arthritis; dermatological diseases such as rheumatoid nodule, skinvasculitis (leukocytoclastic vasculitis), and ischemic skin ulcer; and vascular diseases such as anemia (microcytic hypochromic anemia), splenomegaly, and Felty syndrome involving low white blood cell levels (only the neutrophils). Common complications include interstitial pneumonia, vasculitis, and Sjogren's syndrome.

### (4) Administration Methods

When the preparation of the present invention is parenterally administered (for example, injections), the effective dose differs depending on such factors as, for example, the extent of rheumatoid arthritis symptoms, age, sex, body weight, and administration route, and is not limited. For example, when the active ingredient is the HLA-G (monomer), the effective single dose may be 100 to 1,000 mg, preferably 450 mg for a 60-kg body-weight adult. In the case of the HLA-G dimer, the effective single dose may be 10 to 100 mg, preferably 45 mg for a 60-kg body-weight adult.

Preferably, the preparation should be given locally in parenteral administration, either subcutaneously or intracutaneously using an injection, as mentioned above. As will be described later in Experiment Examples, the local administration of the preparation of the present invention can reduce joint swelling throughout the four limbs, and the anti-rheumatoid arthritis activity can be exhibited. Further, as will be described in Experiment Examples, the preparation of the present invention has a sustained anti-rheumatoid arthritis activity over extended time periods with a single parenteral administration. It is thus preferable that the preparation of the present invention be locally administered, for example, once in at least two months, preferably once in half a month, when the active ingredient is the HLA-G (monomer). In the case of the HLA-G dimer, the preparation of the present invention is preferably given locally once in at least three months, preferably once in two months.

When the preparation of the present invention is orally administered, the administration form and the effective dose depend on such factors as administration target, administration route, properties of the preparation, patient conditions, and the judgment of a physician, and are not limited. For example, when the active ingredient is the HLA-G, a single dose may be 100 to 1, 000 mg, preferably 450 mg for a 60-kg body-weight adult. In the case of the HLA-G dimer, a single dose may be 10 to 100 mg, preferably 45 mg for a 60-kg body-weight adult. A wide range of necessary doses may be appropriately set, taking into consideration that the efficacy varies depending on the administration route.

### Examples

The present invention is described in more detail below based on Examples. The present invention is however not limited to the following Examples.

### Preparation Example 1: Preparation of HLA-G (Monomer)

FIG. 1 is a schematic diagram representing the procedure for producing a modified HLA-G gene. The procedure includes two steps. In FIG. 1, (a) represents the base sequence (SEQ ID NO: 2) of the wild-type HLA-G gene, (b) the base sequence (SEQ ID NO: 3) of an artificially synthesized HLA-G gene (hereinafter, "HLA-GEC gene") prepared to include codons suited for expression in *Escherichia coli* by substituting the codons that encode the total amino acids (SEQ ID NO: 1) of the wild-type HLA-G, (c) the base sequence (SEQ ID NO: 6) of a gene (HLA-GQCa gene) obtained by modifying the 5'-end region (a region corresponding to amino acids 1 to 6) of the HLA-GEC gene, (d) the base sequence (SEQ ID NO: 7) of a gene (HLA-GQCb gene) obtained by modifying the 5'-end region (a region corresponding to amino acids 9 to 15) of the HLA-GEC gene, and (e) the base sequence (SEQ ID NO: 8) of a gene (HLA-GQCab gene) obtained by modifying the 5'-end regions (regions corresponding to amino acids 1 to 6 and 9 to 15) of the HLA-GEC gene (see FIG. 2).

The first step is the production of the HLA-GEC gene (b) by total synthesis, followed by the preparation of the HLA-GQCa gene (c), HLA-GQCb gene (d), and HLA-GQCab gene (e) from the HLA-GEC gene (b) by modifying the 5'-end of the HLA-GEC.

### (1) Preparation of HLA-GEC Gene (b)

HLA-GEC gene (b) (SEQ ID NO: 3), designed to include codons suited for expression in *Escherichia coli* by substituting the codons that encode the total amino acids, was obtained by total synthesis without altering the amino acid sequence (SEQ ID NO: 1) of the wild-type HLA-G. Here, the HLA-GEC gene was prepared to include EcoRV site and NdeI site at the 5'-end, and HindIII site at the 3'-end.

The HLA-GEC gene and a pUC57 vector were then cut with restriction enzymes EcoRV (Takara Bio) and HindIII (TOYOBO), and ligated to each other with a T4 DNA ligase (TOYOBO). The ligated plasmid was cut with restriction enzymes Ndel (Takara Bio) and HindIII, and subjected to agarose gel electrophoresis using a 40 mM Tris-acetate buffer that contained 1 mM EDTA. HLA-GEC gene fragments were cut, and extracted and purified with a QIA quick Gel Extraction Kit (Qiagen). In the same manner, a pGMT7 vector was cut with restriction enzymes NdeI and HindIII, and subjected to agarose gel electrophoresis. The DNA fragments of interest were cut, and extracted and purified. The HLA-GEC and pGMT7 vector were ligated to each other with T4 DNA ligase to construct a HLA-GEC-pGMT7 plasmid. The HLA-GEC-pGMT7 plasmid was used to transform an *Escherichia coli* BL21 (DE3) pLysS strain, and the transformant was cultured at 37°C in a 2 × YT medium supplemented with 100 mg/L ampicillin (0.5% sodium chloride, 1.6% tryptone, 1% dry yeast extract; nacalai tesque).

Thereafter, IPTG (1 mM) was added to the culture suspension at OD₆₀₀ = 0.4 to 0.6, and expression was induced at 37°C for 4 to 6 hours. The bacterial liquid culture (1 mL) was centrifuged before the IPTG expression induction and after 4 hours from the IPTG expression induction, and a sample buffer was directly added to the deposited bacteria. The supernatant was subjected to SDS polyacrylamide gel electrophoresis after a 95°C heat treatment.

### (2) Preparation of HLA-GQCa Gene (c) and HLA-GQCb Gene (d)

HLA-GQCa gene (c) (SEQ ID NO: 6) and HLA-GQCb gene (d) (SEQ ID NO: 7) were prepared as follows. First, a PCR buffer (Promega), a deoxyNTP mixture (TOYOBO), a 5' primer (SEQ ID NO: 9 for the HLA-GQCa gene; SEQ ID NO: 10 for the HLA-GQCb gene), a 3' primer (SEQ ID NO: 11 for the HLA-GQCa gene; SEQ ID NO: 12 for the HLA-GQCb gene) (a final concentration of 0.2 µM each), and PfuTurbo DNA Polymerase (Promega) were added, and PCR was performed using the HLA-GEC-pGMT7 plasmid as a template. For the primers, complementary oligonucleotide primers were used, and the reaction was run in 25 cycles consisting of 30-second denature (95°C), 1-minute annealing (60°C), and 8-minute extension (68°C).

After adding DpnI (NEB) to the PCR product, a reaction was performed at 37°C for 1 hour. After removing the template, the presence of the PCR product was checked by agarose gel electrophoresis. The base sequences were confirmed with a DNA sequencer, and a HLA-GQCa-pGMT7 plasmid and a HLA-GQCb-pGMT7 plasmid were obtained.

The plasmid (HLA-GQCa-pGMT7 or HLA-GQCb-pGMT7) was transformed into the *Escherichia coli* BL21 (DE3) pLysS strain, and HLA-G-producing bacteria (*E.Coli*/HLA-GQCa-pGMT7 or *E.Coli*/HLA-GQCb-pGMT7) were obtained. The transformant was cultured at 37°C in a 2 × YT medium (0.5% sodium chloride, 1.6% tryptone, 1% dry yeast extract) supplemented with 100 mg/L ampicillin. Thereafter, IPTG (1 mM) was added to the culture suspension at OD₆₀₀ = 0.4 to 0.6, and expression was induced at 37°C for 4 to 6 hours. The bacterial liquid culture (1 mL) was centrifuged before the IPTG expression induction and after 4 hours from the IPTG expression induction, and a sample buffer was directly added to the deposited bacteria. The supernatant was subjected to SDS polyacrylamide gel electrophoresis after a 95°C heat treatment.

### (3) Preparation of HLA-GQCab Gene (e)

HLA-GQCab gene (e) (SEQ ID NO: 8) was prepared according to the PCR and other procedures used for the preparation of HLA-GQCa and HLA-GQCb, using HLA-GQCa-pGMT7 or HLA-GQCb-pGMT7 as a template. When HLA-GQCa-pGMT7 is used as a template, the oligonucleotides of SEQ ID NOS: 10 and 12 were used as the 5' primer and 3' primer, respectively. For the HLA-GQCb-pGMT7 template, the oligonucleotides of SEQ ID NOS: 9 and 11 were used as the 5' primer and 3' primer, respectively. The resulting HLA-GQCab-pGMT7 was used to create a transformant as above, and the bacteria were cultured in the same manner as above.

Thereafter, IPTG (1 mM) was added to the culture suspension at OD₆₀₀ = 0.4 to 0.6, and expression was induced at 37°C for 4 to 6 hours. The bacterial liquid culture (1 mL) was centrifuged before the IPTG expression induction and after 4 hours from the IPTG expression induction, and a sample buffer was directly added to the deposited bacteria. The supernatant was subjected to SDS polyacrylamide gel electrophoresis after a 95°C heat treatment.

### (4) Confirmation of Expression

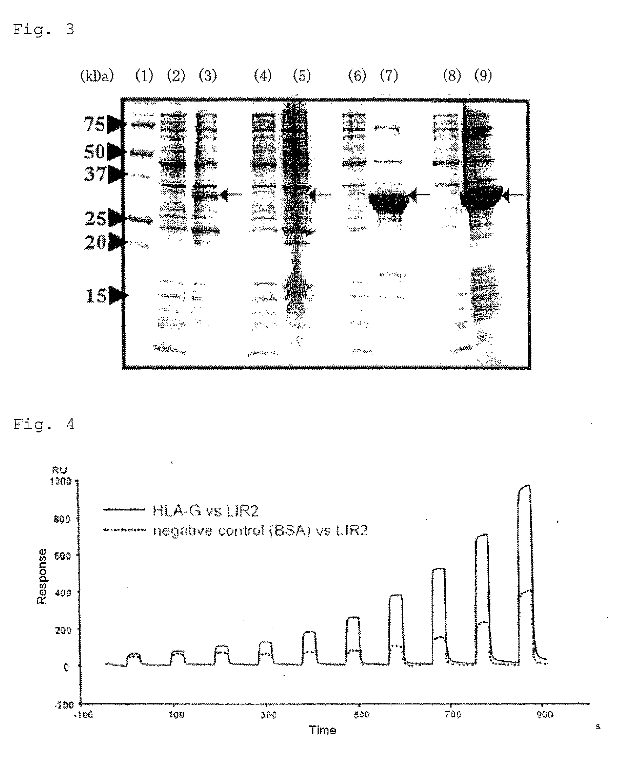

FIG. 3 shows an electrophoresis image representing the expression of the wild-type HLA-G gene (a), HLA-GEC gene (b), HLA-GQCa gene (c), HLA-GQCb gene (d), and HLA-GQCab gene (e). The bacteria used for the SDS polyacrylamide gel electrophoresis were prepared under the same conditions for all genes, and the same amounts were prepared for the electrophoresis.

In FIG. 3, column (1) represents the marker molecular weight. From the marker molecular weight, the arrows in columns (3), (5), (7), and (9) in FIG. 3 indicate the HLA-G molecular weight of about 32 kDa.

In FIG. 3, column (2) represents the electrophoretic pattern of the bacteria before the IPTG expression induction with the wild-type HLA-G gene (SEQ ID NO: 2), and column (3) the electrophoretic pattern of the bacteria after 4 hours from the IPTG expression induction. HLA-G expression is only at the level indicated by arrow in column (3).

In FIG. 3, column (4) represents the electrophoretic pattern of the bacteria before the IPTG expression induction with the HLA-GEC gene (b) (SEQ ID NO: 3), and column (5) the electrophoretic pattern of the bacteria after 4 hours from the IPTG expression induction. No clear band can be recognized at the arrow in column (5), suggesting no HLA-G expression, or very little expression, if any.

In FIG. 3, column (6) represents the electrophoretic pattern of the bacteria before the IPTG expression induction with the HLA-GQCa gene (c) (SEQ ID NO: 6), and column (7) the electrophoretic pattern of the bacteria after 4 hours from the IPTG expression induction. Column (8) represents the electrophoretic pattern of the bacteria before the IPTG expression induction with the HLA-GQCb gene (d) (SEQ ID NO: 7), and column (9) the electrophoretic pattern of the bacteria after 4 hours from the IPTG expression induction. As indicated by arrows in columns (7) and (9), the expression levels in these columns are clearly higher than the expression levels indicated by arrow in columns (3) and (5), in which the wild-type HLA-G gene and the HLA-GEC gene were used, respectively. Further, as clearly indicated by arrow in column (7) that represents expression with HLA-GQCa 4 hours after the IPTG expression induction, more protein is expressed in column (7) than in column (6) that represents expression with HLA-GQCa before the IPTG expression induction. The same can be said for columns (8) and (9) in which HLA-GQCb was used. It was confirmed that the expression levels of HLA-GQCa (c) and HLA-GQCb (d) were much higher than the HLA-GEC gene (b) expression levels, despite that the codons were less frequently used for the HLA-GQCa gene (c) and HLA-GQCb gene (d) in *Escherichia coli,* as represented in FIG. 2.

### (5) Reconstitution and Purification of HLA-G

The bacteria suspension induced to express by addition of IPTG was centrifuged. The bacteria were collected, and suspended in the resuspension buffer (50 mM tris, pH 8.0; 100 mM sodium chloride) added. The bacteria were ultrasonically disrupted, and centrifuged to obtain an inclusion body. The inclusion body was thoroughly washed with Triton wash buffer (0.5% Triton X-100; 50 mM tris, pH 8.0; 100 mM sodium chloride) and resuspension buffer (50 mM tris, pH 8.0; 100 mM sodium chloride), and solubilized with 6.0 M guanidine solution (6.0 M guanidine; 50 mM MES, pH 6.5; 10 mM MEDTA). At this point, the HLA-G solution was measured by ultraviolet absorption. The measured A280 value was about 67, suggesting that the HLA-G expression level was probably about 32 mg/L. Thereafter, human β2 microglobulin (added in 5 times the concentration of HLA-G) and 20 mg of peptides (RIIPRHLQL; SEQ ID NO: 13), which together form a complex with HLA-G, were added. The protein was unwound using a common dilution method, while stirring the suspension at 4°C for 48 hours after adding a refolding buffer (0.1 M tris, pH 8.0; 0.4 M L-arginine; 5 mM EDTA; 3.7 mM cystamine; 6.4 mM cysteamine). The protein was then purified by gel filtration (superdex 75) and ion exchange chromatography (Resource Q).

The HLA-G was then biotinylated by being dissolved in a reaction buffer (50 mM D-biotin, 100 mM ATP, 15 µM BirA) at a concentration of 15 µM. The biotinylated HLA-G and the reaction buffer were separated by gel filtration (Superdex 75) for purification.

### (6) Activity Measurement

Surface plasmon resonance experiment was conducted for HLA-G and LILRB2 (LIR2), using BIAcore 2000^{®} (BIAcore AB, St Albans, England). First, streptavidin was covalently immobilized on a laboratory sensor chip CM5 (BIAcore AB), and the biotinylated HLA-G and the negative control BSA were immobilized via the streptavidin. Then, LILRB2 (LIR2) dissolved in the running buffer HBS-EP (10 mM HEPES, pH 7.5, 150 mM) sodium chloride, 3.4 mM EDTA, 0.005% Surfactant P20) was flown at 10 µL/min. The binding response at each concentration was calculated by subtracting the measured response in a control flow cell from the response in a sample flow cell. Binding constant (Kd) was obtained by Scatchard analysis, or by the nonlinear curve fitting of standard Langmuir binding isotherm, using ORIGIN 5 (Microcal Software).

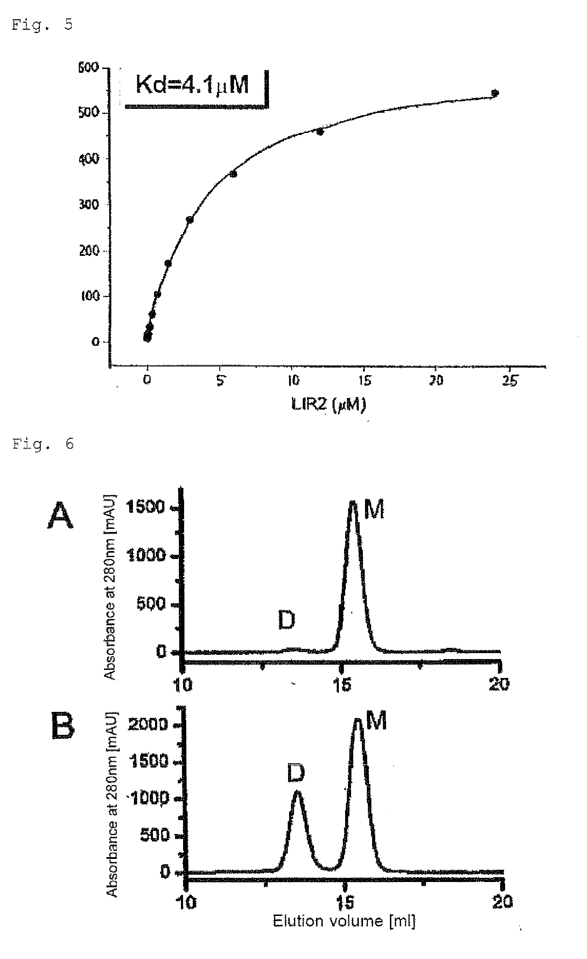

FIG. 4 is a diagram representing the LILRB2 (LIR2) response to the HLA-G or the negative control BSA. The solid line represents the HLA-G immobilized on the sensor chip, and the dotted line represents the BSA immobilized on the sensor chip. As can be seen in FIG. 4, LILRB2 (LIR2) binds more to the HLA-G than to the BSA. FIG. 5 is a diagram representing the Kd value of HLA-G and LILRB2 (LIR2). In FIG. 5, the Kd value is 4.1 µM. This coincides with the result of the HLA-G and LILRB2 (LIR2) surface plasmon resonance experiment for HLA-G expression with the wild-type HLA-G gene (Shiroishi et al., (2003) Proc. Natl. Acad. Sci., USA; ILT4D1D2 in the paper is the same as LILRB2 (LIR2)).

### Preparation Example 2: Preparation of Disulfide-Linked HLA-G Dimer

The HLA-G (monomer) prepared according to the method of Preparation Example 1 was refolded using a refolding buffer (0.1 M Tris-HCl (pH 8.0), 0.4 4 M L-arginine, 3.7 mM cystamine, 6.4 mM cysteamine, 5 mM EDTA) (M. Shiroishi, et al., (2003) Proc. Natl. Acad. Sci., U S A, 100,8856-61.). The HLA-G was then purified by gel filtration (Superdex 75) and ion exchange chromatography (Resource Q). The purified HLA-G was concentrated to 10 mg/mL, and, after 3 to 4 hours, dithiothreitol (DTT) was added at a concentration of 5 mM. The HLA-G was then incubated on ice. An initial crystallization test was performed with the Hydra plus one robot for crystallization, and the INTELLI-PLATE (HAMPTON RESEARCH), using Crystal Screen 1 & 2 (Hampton Research), and Wizard I & II (Art Bobbins). Rod-shaped crystals suited for data collection were obtained after 6 days at 20°C under the conditions of Wizard II No. 39 (0.1 M CAPS (pH 10.5), 20% PEG8000, 0.2 M NaCl).

X-ray diffraction data were collected with the beam line BL38B1 at Spring 8 (Harima, Japan). Prior to data collection, the crystals were immersed in an antifreeze solution (0.1 M CAPS (pH 10.5), 20% PEG8000, 200 mM NaCl, 20% glycerol) and flash-cooled. 3.2 Å diffraction data were obtained at an X-ray wavelength of 1.0000 Å using the ADSC Qauntum 4RCCD system at 100 K. The diffraction data were processed with the HKL-2000 program package, and scaled. The crystals had unit lattice dimensions a = 94.70 Å, b = 127.85 Å, c = 72.60 Å, and belonged to the orthorhombic space group P2₁2₁2. A collection data summary is presented in Table 1. The crystalline structure was revealed by molecular replacement. A clear electron density map observed for the intermolecular Cys42-Cys42 disulfide bond confirmed the crystallization of the HLA-G (wild-type HLA-G) disulfide-linked dimer under these conditions.

**Table 1**

| Collection data summary for HLA-G dimer | |
|---|---|
| | HLA-G dimer |
| PDB ID | 2D31 |
| Data collection | BL3881 (SPring-8) |
| Wavelength (Å) | 1.0000 |
| Resolution range (Å) | 50-3.20 (3.31-3.20) |
| Space group | P2₁2₁2 |
| Unit lattice parameter (Å) | a = 94.70, b = 127.85, c = 72.60 |
| Number of unique reflections | 12,897 (1,162) |
| Completeness (%) | 83.8 (77.7) |
| Multiplicity | 4.9 (4.0) |
| Mean *IIs* (*I*) | 6.2 (2.1) |
| Rmerge^{a} | 0.090 (0.325) |

Conditions for forming a dimer were examined to specify factors that help form the HLA-G disulfide-linked dimer in the crystallization drop. The HLA-G monomer (buffer; 20 mM Tris-HCl (pH 8.0), 100 mM NaCI) concentrated to 10 mg/mL was incubated either at 4°C for 3 days with 2 mM DTT, or for 10 days without DTT. A gel filtration analysis of these mixtures revealed that the addition of DTT significantly promotes HLA-G dimer formation (FIG. 6, A and B).

Thereafter, the amounts of free thiol group in the HLA-G monomer were measured using 5,5'-dithio-bis-2-nitrobenzoate, in order to find whether the free cysteine of the unwound HLA-G molecules was protected. Despite the recent crystalline structure of a HLA-G Cys42Ser mutant suggesting that the Cys42 is completely exposed to solvent (C. S. Clemems, et al., (2005) Proc. Natl. Acad. Sci., USA, 102, 3360-5), only unpaired thiol groups (less than 10%) were detected. This suggests that the most of the free thiol groups in the unwound HLA-G are protected by the reducing agent, for example, cysteamine, contained in the refolding solution. High pH (CAPS pH 10.5) was only slightly effective for dimer formation.

These results suggest that low-concentration DTT promotes formation of the disulfide bonds by attacking the free thiol groups protected by the reducing agent.

### Experiment Example 1

### (1) Creation of Type-II Collagen-Induced Rheumatoid Arthritis Model Mice

### (1-1) Preparation of Bovine Type-II Collagen-Containing Emulsion

A 0.02 M Tris-HCl, 0.15 M NaCl (pH 8.0) buffer was added to bovine type-II collagen, and the collagen was dissolved therein overnight at 4°C under no light. For initial sensitization, an equal amount of Complete Freund's Adjuvant was added, and mixed with a homogenizer for at least 5 min to prepare a bovine type-II collagen-containing emulsion. 100 µg of bovine type-II collagen is contained per 50-µl emulsion/(a dose per mouse). For additional sensitization, an equal amount of Incomplete Freund's Adjuvant was added, and mixed with a homogenizer for at least 5 min to prepare a bovine type-II collagen-containing emulsion. 200 µg of bovine type-II collagen is contained per 50-µl emulsion/(a dose per mouse).

### (1-2) Creation of Type-II Collagen-Induced Rheumatoid Arthritis Model Mice

### (i) Initial Sensitization

52 mice (DBA/1J, male, 6 weeks of age, Charles River) were anesthetized with diethyl ether. With each mouse placed in a mouse holder, 50 µl of the bovine type-II collagen-containing emulsion prepared above was injected into the skin on the base of the tail about 3 cm from the tail base.

### (ii) Additional Sensitization

Two weeks after the initial sensitization, the 52 mice were anesthetized with diethyl ether. With each mouse placed in a mouse holder, 50 µl of the bovine type-II collagen-containing emulsion was injected into the skin on the back of the tail base.

### (iii) Assessment of Type-II Collagen-Induced Rheumatoid Arthritis Model Mice

After additional sensitization, the RA scores of the four limbs were observed and recorded daily (from Monday to Friday), and mice that had about the same scores (type-II collagen-induced rheumatoid arthritis model mice) were checked.

Mice (14 individuals) that had an RA score of 3 after 13 days from the additional sensitization were grouped into a "rheumatoid arthritis early onset group", and mice (14 individuals) that had an RA score of 3 or 4 after 28 days from the additional sensitization were grouped into a "rheumatoid arthritis late onset group". The following experiments were performed for these two groups.

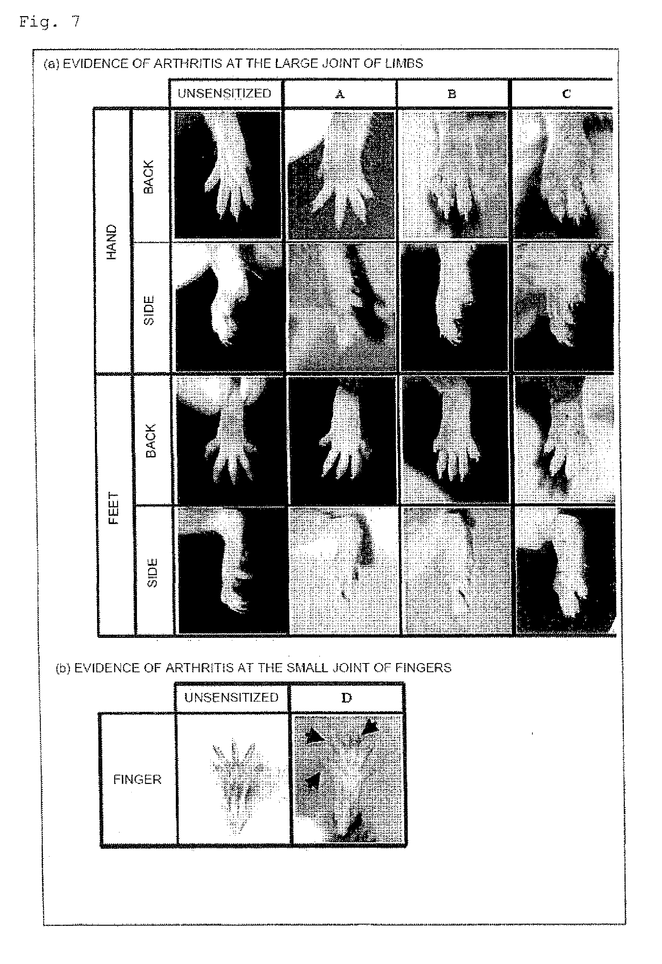

The RA scores were given according to the criteria presented in Table 2, based on (a) the evidence of arthritis at the large joint of limbs (see FIG. 7(a)), and (b) the evidence of arthritis at the small joint of fingers (see FIG. 7 (b)). The total score was then used as the RA score of the rheumatoid arthritis model mouse of interest. Specifically, scores were given based on swelling at the back of the limbs (evidence A = 3 points, evidence B = 4 points, evidence C = 5 points), and swelling at the finger joint (1 point for each finger). More specifically, a maximum of 10 points (a maximum of 5 points for the swelling on the back of a limb + a maximum of 5 points for the swelling at the finger joints) are scored for each limb, and a maximum of 40 points in total for the four limbs. The evaluation and scoring were made by a specified single observer in a blind trial.

**Table 2**

| Evaluation of arthritis at the large joint of limbs | | | |
|---|---|---|---|
| Evidence | | FIG. 7(a) | Score |
| Rubor in one of the limbs | | Evidence A | 3 |
| Rubor and tumor throughout one of the limbs | | Evidence B | 4 |
| Maximum rubor and tumor throughout one of the limbs | | Evidence C | 5 |

| Evaluation of arthritis at the small joint of fingers | | | |
|---|---|---|---|
| Evidence | FIG. 7(b) | Score | |
| Tumor in one of the fingers (a maximum of five in each limb) | Evidence D | 1 for each finger | |

### (2) Confirmation of Rheumatoid Arthritis Therapeutic Effect

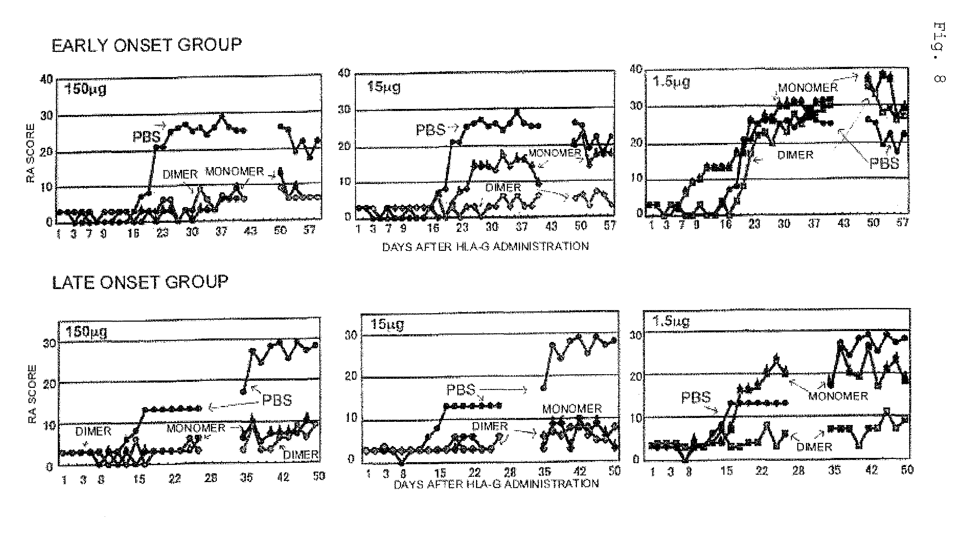

The rheumatoid arthritis early onset group (n = 14), and the rheumatoid arthritis late onset group (n = 14) prepared above were administered with a solution (PBS) that contained the HLA-G monomer (1.5 µg, 15 µg. 150 µg/mouse) prepared in Preparation Example 1, a solution (PBS) that contained the HLA-G dimer (1.5 µg, 15 µg, 150 µg/mouse) prepared in Preparation Example 2, and a control (PBS solution). These solutions were administered intracutaneously from the left foot joint toward the toe, using a 1-ml volume syringe with a 30 G needle. Administration was made after 13 days from additional sensitization for the rheumatoid arthritis early onset group, and on day 28 post additional sensitization for the rheumatoid arthritis late onset group. The mice were observed daily after the administration, and RA scores were recorded for the four limbs (in a blind trial). FIG. 8 represents the experiment results for the rheumatoid arthritis early onset group (n = 14) and the rheumatoid arthritis late onset group (n = 14).

As can be seen in FIG. 8, swelling was observed and there was no suppressing effect in both the control (PBS) group and the rheumatoid arthritis early onset group administered with the HLA-G monomer and HLA-G dimer in 1.5 µg/mouse. It was found, however, that the administration of the HLA-G monomer and HLA-G dimer in 15 µg/mouse suppressed joint swelling, and that the dimer had a stronger and longer suppressing effect than the monomer. It was also confirmed that the administration of the HLA-G monomer and dimer in 150 µg/mouse was able to suppress joint swelling more effectively compared to the control (PBS).

The results thus demonstrated that the HLA-G single administration can sufficiently suppress joint swelling at a single dose of 15 µg for the dimer and 150 µg for the monomer in the rheumatoid arthritis early onset group, and that the effect can last at least about two months.

Further, as can be seen in FIG. 8, the HLA**-**G monomer and HLA-G dimer suppressed joint swelling more effectively than the control (PBS) at doses of 150 µg/mouse and 15 µg/mouse in the rheumatoid arthritis late onset group. The HLA-G dimer had a joint swelling suppressing effect at a dose of 1.5 µg/mouse, whereas the HLA-G monomer did not show suppressing effect at the same dose as evidenced by the presence of joint swelling as in the control.

The results thus demonstrated that the HLA-G single administration can sufficiently suppress joint swelling at a single dose of 1.5 µg for the dimer and 15 µg for the monomer in the rheumatoid arthritis late onset group, and that the effect can last at least 50 days.

Taken together, it was found that the effective HLA-G dose for suppressing joint swelling was different for the rheumatoid arthritis early onset group (presumably high disease susceptibility) and the rheumatoid arthritis late onset group (presumably low disease susceptibility), and that less HLA-G was needed for low disease susceptibility. It was also found that the HLA-G dimer could provide a longer effect than the monomer with a single HLA-G dose at least several-fold lower than that of the monomer. The experiments also confirmed that the local intracutaneous administration (local administration) of the HLA-G monomer and HLA-G dimer to a foot could suppress joint swelling in all of the four limbs.

### Experiment Example 2

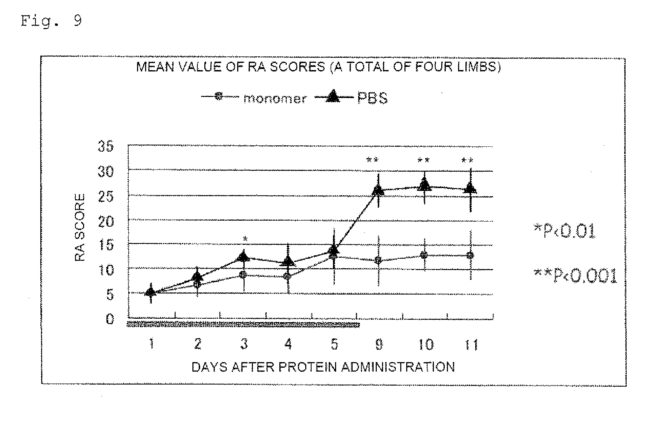

The rheumatoid arthritis onset group created in the same manner as in Experiment Example 1 was divided into two groups (ten mice each). A solution (PBS) containing the HLA-G monomer (35 µg/mouse) prepared in Preparation Example 1, or a control (PBS solution) was then intracutaneously administered from the left foot joint toward the toe, using a 1-ml volume syringe with a 30 G needle. The administration was started on day 13 post additional sensitization, and continued for the next 4 days, once a day. The rheumatoid arthritis onset group was observed daily until day 11 from the start of the administration, and RA scores were recorded for the four limbs (in a blind trial). FIG. 9 represents the RA scores recorded for 11 days from the start of administration for the HLA-G monomer-administered group and for the control (PBS solution) group.

As can be seen from the results, the 5-day successive daily administration of the HLA-G monomer suppressed joint swelling, and the joint swelling suppressing effect lasted even after 5 days from the HLA-G monomer administration. It can be said from these results that the single administration of the HLA-G monomer can provide a persistent joint-swelling suppressing effect (rheumatoid arthritis therapeutic effect). This was demonstrated in Experiment Example 1, and is supported by the results.

### Control Experiment Example 1

The HLA-G monomer and HLA-G dimer were applied to the auricle of the both ears of mice sensitized with an Ascaris extract (Ascaris antigens) to study the effect on immediate or late-onset dermatitis (Ascaris antigen-induced dermatitis).

### Test Methods

BALB/cAnNCrlCrlj mice (male), 7 weeks of age (Charles River) were sensitized by the intraperitoneal administration of a 0.5-ml mixture of 800 µg/ml DNP-Ascaris and an equal amount of an aluminum hydroxide gel suspension. After 13 days from the sensitization, the mice were grouped (control (PBS) group, HLA-G monomer group, HLA-G dimer group) substantially uniformly based on average body weight. After 14 days from the sensitization, a DNP-Ascaris solution adjusted to 1 mg/ml with a physiological saline was administered into the skin on the inner side of the auricle of the both ears (0.01 ml each) to induce dermatitis.

PBS, HLA-G monomer (160 µg/ml), and HLA-G dimer (16 µg/ml) were applied twice to the skin on the inner side of the auricle of the both ears (0.02 ml to each ear) 3 hours before the induction and 4 hours after the induction. The auricle thickness was measured for the both ears on the day before the induction, and after 1, 4, 24, and 48 hours from the induction, once each time. An increase in auricle thickness was then calculated by subtracting the pre-induction thickness from each measured thickness after the induction. The body weight was also measured after 13 days from the sensitization, and after 48 hours of observation from the induction.

### Results

There was no statistically significant difference in auricle thickness (mean value) between the HLA-G dimer and the control group; however, the thickness values after 1 hour and 4 hours were smaller than the thicknesses measured after 24, and 48 hours. The results from the right ears yielded significantly low values 4 hours post induction. On the other hand, the auricle thickness (mean value) after 1 hour from the induction had statistically significant low values in the HLA-G monomer compared to the control group. The values remained low after 4 hours, though not significant; however, no effect was observed after 24, and 48 hours.

The HLA-G monomer and dimer produced no side effects attributed to the HLA-G administration, neither in the common conditions and the body weight of the mice.

As demonstrated above, the dermatitis suppressing effect was observed only in relatively short time periods from the induction (1 hour or 4 hours), and diminished by 24 and 48 hours from the induction.

This result suggests that the inflammation suppressing effect of HLA-G (HLA-G monomer, dimer) for the Ascaris antigen-induced dermatitis model animals is brief, and that multiple, continuous administration is needed in mice to sustain the effect. However, contrary to what is expected from this result, the inflammation suppressing effect of HLA-G (HLA-G monomer, dimer) was found to be long lasting, at least 50 days with a single administration, in the rheumatoid arthritis model animals, as demonstrated in Experiment Examples 1 and 2.

### Sequence Listing Free Text

SEQ ID NO: 3 represents the base sequence of the HLA-G gene modified from the base sequence of the wild-type HLA-G gene to be suited for expression in *Escherichia coli.* SEQ ID NO: 4 is the base sequence used to substitute the base sequence from positions 1 to 18 of SEQ ID NO: 3; SEQ ID NO: 5 the base sequence used to substitute the base sequence from positions 25 to 45 of SEQ ID NO: 3; SEQ ID NO: 6 the base sequence including the base sequence of SEQ ID NO: 4 substituting the base sequence from positions 1 to 18 of SEQ ID NO: 3; SEQ ID NO: 7 the base sequence including the base sequence of SEQ ID NO: 5 substituting the base sequence from positions 25 to 45 of SEQ ID NO: 3; SEQ ID NO: 8 the base sequence including the base sequence of SEQ ID NO: 4 substituting the base sequence from positions 1 to 18 of SEQ ID NO: 3, and the base sequence of SEQ ID NO: 5 substituting the base sequence from positions 25 to 45 of SEQ ID NO: 3.

SEQ ID NOS: 9 and 11 represent the base sequences of the 5' primer and 3' primer, respectively, used for PCR that uses HLA-GQCb-pGMT7 as a template. SEQ ID NOS: 10 and 12 represent the base sequences of the 5' primer and 3' primer, respectively, used for PCR that uses HLA-GQCa-pGMT7 as a template. SEQ ID NO: 13 represents the amino acid sequence of the peptides used in Preparation Example 1 (5).

SEQUENCE LISTING

<110>  KYUSHU UNIVERSITY, NATIONAL UNIVERSITY CORPORATION

<120>  Prophylactic or therapeutic agent for theumatoid arthritis or
rheumatoid arthritis-related diseases

<130>  T2756 EP S3

<140>  EP 10 74 8786.0
<141>  2010-03-03

<150>  JP2009-049792
<151>  2009-03-03

<160>  13

<170>  PatentIn version 3.3

<210>  1
<211>  276
<212>  PRT
<213>  Homo sapiens
<400>  1
Gly Ser His Ser Met Arg Tyr Phe Ser Ala Ala Val Ser Arg Pro Gly
1               5                   10                  15
Arg Gly Glu Pro Arg Phe Ile Ala Met Gly Tyr Val Asp Asp Thr Gln
            20                  25                  30
Phe Val Arg Phe Asp Ser Asp Ser Ala Cys Pro Arg Met Glu Pro Arg
        35                  40                  45
Ala Pro Trp Val Glu Gln Glu Gly Pro Glu Tyr Trp Glu Glu Glu Thr
    50                  55                  60
Arg Asn Thr Lys Ala His Ala Gln Thr Asp Arg Met Asn Leu Gln Thr
65                  70                  75                  80
Leu Arg Gly Tyr Tyr Asn Gln Ser Glu Ala Ser Ser His Thr Leu Gln
                85                  90                  95
Trp Met Ile Gly Cys Asp Leu Gly Ser Asp Gly Arg Leu Leu Arg Gly
            100                 105                 110
Tyr Glu Gln Tyr Ala Tyr Asp Gly Lys Asp Tyr Leu Ala Leu Asn Glu
        115                 120                 125
Asp Leu Arg Ser Trp Thr Ala Ala Asp Thr Ala Ala Gln Ile Ser Lys
    130                 135                 140
Arg Lys Cys Glu Ala Ala Asn Val Ala Glu Gln Arg Arg Ala Tyr Leu
145                 150                 155                 160
Glu Gly Thr Cys Val Glu Trp Leu His Arg Tyr Leu Glu Asn Gly Lys
                165                 170                 175
Glu Met Leu Gln Arg Ala Asp Pro Pro Lys Thr His Val Thr His His
            180                 185                 190
Pro Val Phe Asp Tyr Glu Ala Thr Leu Arg Cys Trp Ala Leu Gly Phe
        195                 200                 205
Tyr Pro Ala Glu Ile Ile Leu Thr Trp Gln Arg Asp Gly Glu Asp Gln
    210                 215                 220
Thr Gln Asp Val Glu Leu Val Glu Thr Arg Pro Ala Gly Asp Gly Thr
225                 230                 235                 240
Phe Gln Lys Trp Ala Ala Val Val Val Pro Ser Gly Glu Glu Gln Arg
                245                 250                 255
Tyr Thr Cys His Val Gln His Glu Gly Leu Pro Glu Pro Leu Met Leu
            260                 265                 270
Arg Trp Lys Gln
        275

<210> 2
<211> 828
<212> DNA
<213> Homo sapiens
<400> 2
ggctcccact ccatgaggta tttcagcgcc gccgtgtccc ggcccggccg cggggagccc 60
cgcttcatcg ccatgggcta cgtggacgac acgcagttcg tgcggttcga cagcgactcg 120
gcgtgtccga ggatggagcc gcgggcgccg tgggtggagc aggaggggcc agagtattgg 180
gaagaggaga cacggaacac caaggcccac gcacagactg acagaatgaa cctgcagacc 240
ctgcgcggct actacaacca gagcgaggcc agttctcaca ccctccagtg gatgattggc 300
tgcgacctgg ggtccgacgg acgcctcatc cgcgggtatg aacggtatgc ctacgatggc 360
aaggattacc tcgccctgaa cgaggacctg cgctcctgga ccgcagcgga cactgcggct 420
cagatctcca agcgcaagtg tgaggcggcc aatgtggctg aacaaaggag agcctacctg 480
gagggcacgt gcgtggagtg gctccacaga tacctggaga acgggaagga gatgctgcag 540
cgcgcggacc cccccaagac acacgtgacc caccaccctg tctttgacta tgaggccacc 600
ctgaggtgct gggccctggg cttctaccct gcggagatca tactgacctg gcagcgggat 660
ggggaggacc agacccagga cgtggagctc gtggagacca ggcctgcagg ggatggaacc 720
ttccagaagt gggcagctgt ggtggtgcct tctggagagg agcagagata cacgtgccat 780
gtgcagcatg aggggctgcc ggagcccctc atgctgagat ggaagcag 828

<210> 3
<211> 828
<212> DNA
<213> Artificial Sequence
<220>
<223> Base Sequence of modified HLA-G gene, wherein codon sequence is
altered to be expressed in E.coli.
<400> 3
ggcagccaca gcatgcgcta ttttagcgcg gccgtgagcc gtccgggccg cggcgaaccg 60
cgctttatcg cgatgggcta tgtggatgat acccagttcg tgcgctttga tagcgatagc 120
gcgtgcccgc gtatggaacc gcgtgccccg tgggttgaac aggaaggtcc ggaatattgg 180
gaagaagaaa cccgcaacac caaagcccat gcgcagaccg atcgtatgaa cctgcagacc 240
ctgcgcggct attataacca gagcgaagcg agcagccata ccctgcagtg gatgattggt 300
tgtgatctgg gcagcgatgg ccgtctgctg cgtggttatg aacagtatgc ctatgatggc 360
aaagattatc tggcgctgaa tgaagatctg cgtagctgga ccgccgcgga taccgcggcg 420
cagattagca aacgtaaatg cgaagcggcc aacgtggcgg aacagcgccg cgcgtatctg 480
gaaggtacct gtgttgaatg gctgcatcgc tatctggaaa atggtaaaga aatgctgcag 540
cgtgccgatc cgccgaaaac ccatgttacc catcatccgg tgttcgatta tgaagcgacc 600
ctgcgctgct gggccctggg cttttatccg gccgaaatta ttctgacctg gcagcgtgat 660
ggcgaagatc agacccagga tgtggaactg gtggaaaccc gtccggcggg tgatggtacc 720
tttcagaaat gggcggcggt tgtggtgccg agcggcgaag aacagcgcta tacctgccat 780
gtgcagcatg aaggtctgcc ggaaccgctg atgctgcgct ggaaacag 828

<210> 4
<211> 18
<212> DNA
<213> Artificial Sequence
<220>
<223> Sequence of 1-18 region of Base Sequence of modified HLA-G gene,
wherein codon sequence is altered to be expressed in E.coli.
<400> 4
ggtagtcata gtatgcgt 18

<210> 5
<211> 21
<212> DNA
<213> Artificial Sequence
<220>
<223> Sequence of 25-45 region of Base Sequence of modified HLA-G gene,

wherein codon sequence is altered to be expressed in E.coli.
<400> 5
agtgcagcag taagtcgtcc a 21

<210> 6
<211> 828
<212> DNA
<213> Artificial Sequence
<220>
<223> Base Sequence of modified HLA-G gene (HLA-GQCa gene)
<400> 6
ggtagtcata gtatgcgtta ttttagcgcg gccgtgagcc gtccgggccg cggcgaaccg 60
cgctttatcg cgatgggcta tgtggatgat acccagttcg tgcgctttga tagcgatagc 120
gcgtgcccgc gtatggaacc gcgtgccccg tgggttgaac aggaaggtcc ggaatattgg 180
gaagaagaaa cccgcaacac caaagcccat gcgcagaccg atcgtatgaa cctgcagacc 240
ctgcgcggct attataacca gagcgaagcg agcagccata ccctgcagtg gatgattggt 300
tgtgatctgg gcagcgatgg ccgtctgctg cgtggttatg aacagtatgc ctatgatggc 360
aaagattatc tggcgctgaa tgaagatctg cgtagctgga ccgccgcgga taccgcggcg 420
cagattagca aacgtaaatg cgaagcggcc aacgtggcgg aacagcgccg cgcgtatctg 480
gaaggtacct gtgttgaatg gctgcatcgc tatctggaaa atggtaaaga aatgctgcag 540
cgtgccgatc cgccgaaaac ccatgttacc catcatccgg tgttcgatta tgaagcgacc 600
ctgcgctgct gggccctggg cttttatccg gccgaaatta ttctgacctg gcagcgtgat 660
ggcgaagatc agacccagga tgtggaactg gtggaaaccc gtccggcggg tgatggtacc 720
tttcagaaat gggcggcggt tgtggtgccg agcggcgaag aacagcgcta tacctgccat 780
gtgcagcatg aaggtctgcc ggaaccgctg atgctgcgct ggaaacag 828

<210> 7
<211> 828
<212> DNA
<213> Artificial Sequence
<220>
<223> Base Sequence of modified HLA-G gene (HLA-GQCb gene)
<400> 7
ggcagccaca gcatgcgcta ttttagtgca gcagtaagtc gtccaggccg cggcgaaccg 60
cgctttatcg cgatgggcta tgtggatgat acccagttcg tgcgctttga tagcgatagc 120
gcgtgcccgc gtatggaacc gcgtgccccg tgggttgaac aggaaggtcc ggaatattgg 180
gaagaagaaa cccgcaacac caaagcccat gcgcagaccg atcgtatgaa cctgcagacc 240
ctgcgcggct attataacca gagcgaagcg agcagccata ccctgcagtg gatgattggt 300
tgtgatctgg gcagcgatgg ccgtctgctg cgtggttatg aacagtatgc ctatgatggc 360
aaagattatc tggcgctgaa tgaagatctg cgtagctgga ccgccgcgga taccgcggcg 420
cagattagca aacgtaaatg cgaagcggcc aacgtggcgg aacagcgccg cgcgtatctg 480
gaaggtacct gtgttgaatg gctgcatcgc tatctggaaa atggtaaaga aatgctgcag 540
cgtgccgatc cgccgaaaac ccatgttacc catcatccgg tgttcgatta tgaagcgacc 600
ctgcgctgct gggccctggg cttttatccg gccgaaatta ttctgacctg gcagcgtgat 660
ggcgaagatc agacccagga tgtggaactg gtggaaaccc gtccggcggg tgatggtacc 720
tttcagaaat gggcggcggt tgtggtgccg agcggcgaag aacagcgcta tacctgccat 780
gtgcagcatg aaggtctgcc ggaaccgctg atgctgcgct ggaaacag 828

<210> 8
<211> 828
<212> DNA
<213> Artificial Sequence
<220>
<223> Base Sequence of modified HLA-G gene (HLA-GQCab gene)
<400> 8
ggtagtcata gtatgcgtta ttttagtgca gcagtaagtc gtccaggccg cggcgaaccg 60
cgctttatcg cgatgggcta tgtggatgat acccagttcg tgcgctttga tagcgatagc 120
gcgtgcccgc gtatggaacc gcgtgccccg tgggttgaac aggaaggtcc ggaatattgg 180
gaagaagaaa cccgcaacac caaagcccat gcgcagaccg atcgtatgaa cctgcagacc 240

ctgcgcggct attataacca gagcgaagcg agcagccata ccctgcagtg gatgattggt 300
tgtgatctgg gcagcgatgg ccgtctgctg cgtggttatg aacagtatgc ctatgatggc 360
aaagattatc tggcgctgaa tgaagatctg cgtagctgga ccgccgcgga taccgcggcg 420
cagattagca aacgtaaatg cgaagcggcc aacgtggcgg aacagcgccg cgcgtatctg 480
gaaggtacct gtgttgaatg gctgcatcgc tatctggaaa atggtaaaga aatgctgcag 540
cgtgccgatc cgccgaaaac ccatgttacc catcatccgg tgttcgatta tgaagcgacc 600
ctgcgctgct gggccctggg cttttatccg gccgaaatta ttctgacctg gcagcgtgat 660
ggcgaagatc agacccagga tgtggaactg gtggaaaccc gtccggcggg tgatggtacc 720
tttcagaaat gggcggcggt tgtggtgccg agcggcgaag aacagcgcta tacctgccat 780
gtgcagcatg aaggtctgcc ggaaccgctg atgctgcgct ggaaacag
\201@\201@ 828

<210> 9
<211> 41
<212> DNA
<213> Artificial Sequence
<220>
<223> Sequence of 5\201fprimer for PCR using HLA-GQCb-pGMT7 as a template
<400> 9
atgggtagtc atagtatgcg ttattttagc gcggccgtga g 41

<210> 10
<211> 42
<212> DNA
<213> Artificial Sequence
<220>
<223> Sequence of 5\201fprimer for PCR using HLA-GQCa-pGMT7 as a template
<400> 10
cgctatttta gtgcagcagt aagtcgtcca ggccgcggcg aa 42

<210> 11
<211> 41
<212> DNA
<213> Artificial Sequence
<220>
<223> Sequence of 3\201fprimer for PCR using HLA-GQCb-pGMT7 as a template
<400> 11
ctcacggccg cgctaaaata acgcatacta tgactaccca t 41

<210> 12
<211> 42
<212> DNA
<213> Artificial Sequence
<220>
<223> Sequence of 3\201fprimer for PCR using HLA-GQCa-pGMT7 as a template
<400> 12
ttcgccgcgg cctggacgac ttactgctgc actaaaatag cg 42

<210> 13
<211> 9
<212> PRT
<213> Artificial Sequence
<220>
<223> Peptide
<400> 13
Arg Ile Ile Pro Arg His Leu Gln Leu
                5

## Claims

1. A prophylactic or therapeutic agent for rheumatoid arthritis or for a disease caused by rheumatoid arthritis, comprising a HLA-G or a dimer thereof as an active ingredient.

2. The prophylactic or therapeutic agent according to claim 1, wherein the HLA-G is a protein of (a) or (b) below:
(a) a protein consisting of the amino acid sequence of SEQ ID NO: 1,
(b) a protein consisting of the amino acid sequence of SEQ ID NO: 1 with the deletion, substitution, or addition of one or several amino acids excluding the amino acid at position 42, and having a binding activity for leukocyte Ig-like receptors and/or CD8.

3. The prophylactic or therapeutic agent of claim 1 or 2,
wherein the HLA-G dimer is a dimer with an intermolecular disulfide bond that links the HLA-G between cysteine residues at position 42 of a HLA-G amino acid sequence.

4. The prophylactic or therapeutic agent according to claim 1 or 2, wherein the prophylactic or therapeutic agent is a preparation that includes the HLA-G (monomer) as an active ingredient, and that is of a form locally administered to a patient with rheumatoid arthritis or with a disease caused by rheumatoid arthritis, or to a patient having the possibility of developing the disease, once in at least two months.

5. The prophylactic or therapeutic agent according to claim 1 or 2, wherein the prophylactic or therapeutic agent is a preparation that includes the HLA-G dimer as an active ingredient, and that is of a form locally administered to a patient with rheumatoid arthritis or with a disease caused by rheumatoid arthritis, or to a patient having the possibility of developing the disease, once at least in three months.

6. A method for preventing or treating rheumatoid arthritis or a disease caused by rheumatoid arthritis,
comprising the step of administering an effective amount of a composition that includes a HLA-G or a dimer thereof as an active ingredient to a patient with rheumatoid arthritis or with a disease caused by rheumatoid arthritis, or to a patient having the possibility of developing the disease.

7. The method according to claim 6, wherein the HLA-G is a protein of (a) or (b) below:
(a) a protein consisting of the amino acid sequence of SEQ ID NO: 1,
(b) a protein consisting of the amino acid sequence of SEQ ID NO: 1 with the deletion, substitution, or addition of one or several amino acids excluding the amino acid at position 42, and having a binding activity for leukocyte Ig-like receptors and/or CD8.

8. The method according to claim 6 or 7, wherein the HLA-G dimer is a dimer with an intermolecular disulfide bond that links the HLA-G between cysteine residues at position 42 of a HLA-G amino acid sequence.

9. The method according to claim 6 or 7, wherein the composition is a pharmaceutical preparation that includes the HLA-G (monomer) as an active ingredient, and is locally administered to a patient with rheumatoid arthritis or with a disease caused by rheumatoid arthritis, or to a patient having the possibility of developing the disease, once in at least two months.

10. The method according to claim 6 or 7, wherein the composition is a pharmaceutical preparation that includes the HLA-G dimer as an active ingredient, and is locally administered to a patient with rheumatoid arthritis or with a disease caused by rheumatoid arthritis, or to a patient having the possibility of developing the disease, once in at least three months.

11. A HLA-G, a dimer thereof, or a pharmaceutical composition for use in the prevention or treatment of rheumatoid arthritis or a disease caused by rheumatoid arthritis;
the pharmaceutical composition comprising the HLA-G or the HLA-G dimer in an effective proportion for the prevention or treatment of rheumatoid arthritis or a rheumatoid arthritis-related disease.

12. The HLA-G or a dimer thereof, or a pharmaceutical composition comprising the HLA-G or the HLA-G dimer according to claim 11, wherein the HLA-G is a protein of (a) or (b) below.

13. The HLA-G or a dimer thereof, or a pharmaceutical composition comprising the HLA-G or the HLA-G dimer according to claim 11 or 12, wherein the HLA-G dimer is a dimer with an intermolecular disulfide bond that links the HLA-G between cysteine residue at position 42 of a HLA-G amino acid sequence.

14. The HLA-G or a dimer thereof, or a pharmaceutical composition comprising the HLA-G or the HLA-G dimer according to claim 11 or 12, wherein the pharmaceutical composition is a preparation that includes the HLA-G (monomer) as an active ingredient, and that is of a form locally administered to a patient with rheumatoid arthritis or with a disease caused by rheumatoid arthritis, or to a patient having the possibility of developing the disease, once in at least three months.

15. The HLA-G or a dimer thereof, or a pharmaceutical composition comprising the HLA-G or the HLA-G dimer according to claim 11 or 12, wherein the pharmaceutical composition is a preparation that includes the HLA-G dimer as an active ingredient, and that is of a form locally administered to a patient with rheumatoid arthritis or with a disease caused by rheumatoid arthritis, or to a patient having the possibility of developing the disease, once in at least two months.
